# EUROPEAN PATENT APPLICATION

(11) **EP 4 159 267 A1**
(43) Date of publication of application: **05.04.2023**
(21) Application number: 21811941.0
(22) Date of filing: 21.05.2021
(51) Int. Cl.: A61M 39/20, A61M 39/28, A61M 39/02, A61M 39/06

(54) **BARRIER KIT FOR AN ENDOTRACHEAL TUBE WITH SEALING COVER COUPLED TO THE CLAMP WITH VARIOUS STAGES FOR ADJUSTING THE DEGREE OF OCCLUSION FOR USE IN ADVANCED AIRWAY MANAGEMENT PROCEDURES**

(30) Priority: 27.05.2020 BR 102020010672
(71) Applicant: PFD - Comércio Varejista de Equipamentos Médicos Ltda., Porto Alegre, RS CEP 90690-340 (BR)
(72) Inventor: FERREIRA PASETTO, Pedro, 79040-010 Campo Grande (BR); FELIPE CARAMEZ BERTEGES, Luiz, 26700-000 Mendes (BR); MONTEIRO TAVARES PEREIRA, Bruno, 13085-015 Campinas (BR); TAVARES LIMA TRAJANO, Eduardo, 27700-000 Vassouras (BR); SILVEIRA RODRIGUES JÚNIOR, Adauri, 27195-000 Piraí (BR)
(74) Representative: López Camba, Emilia
(86) International application number: PCT/BR2021/050219
(87) International publication number: WO 2021/237325

(57) **Abstract**

The present invention applies to tubes and probes and catheters for invasive airway management, more specifically to a kit comprising two devices for sealing the tubes and/or probes and/or endotracheal catheters for gases, aerosols, droplets and secretions from patients. The present invention describes an Endotracheal Tube Barrier Kit with sealing cap attached to *clamp* for use in advanced airway management procedures consisting of two devices, one for sealing and one for "clamping" the tube, coupled together. The kit consists of the following components: sealing device (1) and *occluder clamp* (2). Illustratively, to understand more clearly the use of the Barrier Kit, the tube devices (3) are also shown, connector (4) and guide wire (5).

## Description

### FIELD OF APPLICATION

The present invention applies to tubes and probes and catheters for invasive airway management, more specifically to a kit comprising two devices for sealing the tubes and/or probes and/or endotracheal catheters for gases, aerosols, droplets and secretions from patients. This kit will contribute to the medicine and safety of health care workers, especially with regard to the Covid-19 pandemic.

### GROUNDS FOR THE INVENTION

A relevant problem in the state of the art is the possibility of contamination of health care workers by aerosol and/or secretions during the tracheal intubation procedure in patients with an indication for invasive airway management.

Furthermore, there is an absence of available devices designed for the purposes of "clamping" and sealing the endotracheal tubes, probes, or catheters that are often readily accessible for these purposes in these situations. The need to use adaptations, improvisations, or materials that are not proper for the activity, allows the occurrence of accidents and injuries to health professionals, or even delays in the assistance to the patient in need due to the lack of readily available material, besides not being the best way, nor the best material, to perform the care.

The present invention inexpensively and simply provides improved safety for health care professionals involved in invasive airway management by avoiding, or significantly minimizing, the possibility of contamination by secretion, droplets and/or aerosols.

The proposed model is easy to transport and adapt to the tubes, which makes it feasible to use from the pre-hospital to the intra-hospital environment, and makes it available in all emergency, operating room, and intensive care rooms, or in any environment where advanced patient airway management is performed. Additionally, another advantage is that this kit can be made from 3D printers.

The *occluder clamp of* the present invention as it is made of plastic material, including poly lactic acid - PLA (biodegradable and compostable, used for 3D printing), can be made on 3D printers, and can be produced anywhere these printers are available.

This device has the potential to create habits, change protocols, or even influence the way endotracheal tubes are produced today, with the possibility of mandatory use of such devices being envisioned in the future.

### TECHNIQUE STATUS

Document EP2736586 describes a holding device for holding at least one tube clamp on a medical tube, wherein the tube clamp preferably has two clamping sections to change the lumen cross-section of the medical tube in use. The pipe clamp has two pressure pieces to close the clamp in use. The holding device also has a receiving frame, which surrounds the pipe, to receive the pipe clamp in a parking position or inclined position. The device has a detachable end cap, which connects to the connection section to prevent contamination of the holding device until the pipe is used. Note that the connection section has a central opening, allowing the tube to be connected, for example, to a patient's arterial or venous access, or to a connecting piece of a medical device, among others.

However, such a device differs from the present invention in that it is visualized that the tube clamp has only two fins, protuberances, or clamping sections, as described, on opposite sides, parallel when closing. The positioning of such fins does not allow for an adequate tube deformation angle, or *kinking.* This type of design works only for liquid sealing. To obtain a complete occlusion, including for gases and aerosols, there is the need for a greater deformity of the tube, which is achieved with the presence of another fin on one of the flaps of the clamp, according to the *occluder* clamp of the present invention, which allows a better fitting of the flaps and, consequently, a better occlusion. Besides this, it is evident that the so-called clamping sections are sharp, which can cause the pipe to fracture, hence the need to use an additional device, called a restraint device, as described in the document.

Additionally, the *occluder clamp* fins of the present invention are rounded, i.e., they are atraumatic and allow for safer "clamping" anywhere on the tube without the need for an additional device.

The tube clamp in this European document has only a single clamping stage, which does not make it possible to adjust the required degree of occlusion and does not allow its use in the various existing tube sizes. Therefore, the present invention differs from this object in that it has several stages of adjustment in the *occluder clamp,* which enables its universal use in endotracheal tubes, as well as the adjustment of the occlusion.

The *occluder clamp* of the present invention also has a fitting for the sealing device, which allows both parts to be sold and transported in a simplified manner, which is not evidenced in the European patent, and its more ergonomic shape, with a fitting for the finger, which is absent in the tube clamp, allows for more comfort and improves the mechanics of its use. The *occluder clamp* is more complex and complete in its engineering, which allows it to function more adequately in the end activity.

In that device in the European document, the holding device is connected to the connection section, which has a detachable cover for the purpose of preventing contamination of that holding device, as described, and not contamination of the pipe. A central opening in the connection section is described for connection to a venous or arterial access (or connection to a medical device), which indicates that this material is not suitable for use in endotracheal tubes, or advanced airway management. This connection is only possible with the lid open, i.e. allowing gases and/or secretions to escape. Neither the hole in the lid nor the malleability of the material is described.

The Sealing Device of the present invention differs from the one disclosed in this document in that it reveals that the sealing lid of the barrier kit is made of flexible material, with the presence of a central virtual hole, corresponding to a weakness of the material in this region, for the passage of the guidewire during the endotracheal intubation procedure, a possibility that is not described in the European patent at any time. Its elastic composition allows the deformation of its body in a way that allows the penetration of the guide wire, of greater thickness, through the virtual orifice, sealing the exit of gases, aerosols, droplets and secretions by the elastic pressure that the material makes against the wall of the guide wire. In addition, there is the presence of an extraction ear, to facilitate the removal of this device. Thus, there is no similarity whatsoever with the cover described in the European document, even with different functions.

The product called "Endotracheal/Orotracheal Tube Clamp - COVID19 Coronavirus covid-19" (available at https://www.thingiverse.com/ thing:4271154) shows that it can be used in orotracheal tubes so that during the medical act of intubation of patients infected with COVID-19 the air passage can be interrupted decreasing the chance of contamination of the health care worker. The product was tested in tubes with the following measures (internal diameter): 7mm; 7.5mm. The *clamp* is a one-piece plastic part with two cylindrical-shaped protuberances on opposite sides. It also has two circular holes that allow the tube to pass through the inside of the clamp, and has ends that allow it to be closed. This clamp is made of PLA (polylactic acid) plastic material, which is flexible.

This product has only two fins, which do not allow for an adequate tube deformation angle, or *kinking,* and therefore should only work for liquid sealing, as this design does not allow for complete sealing for gases and aerosols. The *occluder clamp* has three fins, which allows a better fitting of the fins and, consequently, a better occlusion of the tube. This product has two pressure parts, only a single clamping stage, and is only suitable for use with 7.0 and 7.5mm pipes. The *occluder clamp of* the present invention has several stages of adjustment and can be used on the various existing diameters of endotracheal tubes. In addition, the *occluder clamp* still features a fitting for the sealing device and is more ergonomic, featuring a finger fitting, which allows for more comfort and improves the mechanics of its use. The *occluder clamp* is more complex and complete in its engineering, which allows it to function more adequately in the end activity. The one revealed by this paper has no sealing cap, like the Barrier Kit proposed here. Thus, the one revealed by this document is not characterized by a Barrier Kit for not having the sealing cap, nor even has the same characteristics as the *occluder clamp* , which is more complex, complete, and functional.

Document US7758552 discloses a pipe clamp applied to selectively open or close a pipe. This device is molded as a U-shaped shackle with rigid legs, having projections on its inner surface configured with clamping edges, and because it is made of flexible material it allows the legs to be pulled apart in a divergent position and to be pulled apart from each other. The first leg features at its end a flexible, deflectable spring-loaded latch and a lock, which serves to keep the clamp clamp in the locked position when the legs are pressed together.

The tube clamp disclosed herein differs from the *occluder clamp* of the present invention in that the clamp has only two fins, while the *occluder* clamp has three fins. The tube clamp has two pressure parts and only a single stage of clamping, while the *occluder* clamp has several stages of adjustment. The *occluder clamp* also features a fitting for the sealing device and is more ergonomic, featuring a finger attachment, which allows for more comfort and improves the mechanics of its use. The *occluder clamp* is more complex and complete in its engineering, which allows it to function more adequately in the end activity. The one disclosed by this paper does not have a sealing cap, like the Barrier Kit of the present invention. Thus, the one revealed by this document is not characterized by a Barrier Kit for not having the sealing cap, nor even has the same characteristics as the *occluder clamp*, which is more complex, complete, and functional.

### SUMMARY OF THE INVENTION

The present invention describes an Endotracheal Tube Barrier Kit with sealing cap attached to *clamp* for use in advanced airway management procedures consisting of two devices, one for sealing and one for "clamping" the tube, coupled together. The kit consists of the following components: sealing device (1) and *occluder clamp (2).* Illustratively, to understand more clearly the use of the Barrier Kit, the tube devices (3) are also shown, connector (4) and guide wire (5), which are components to which the barrier kit is applied.

The present invention applies to tubes and probes and catheters for invasive airway management, more specifically to a kit comprising two devices for sealing the tubes and/or probes and/or endotracheal catheters for gases, aerosols, droplets and secretions from patients. This barrier kit will contribute to the medicine and safety of health care workers, especially with regard to the Covid-19 pandemic.

### BRIEF DESCRIPTION OF THE FIGURES

Figure 1 shows the sealing device attached to the *occluder clamp* device.
Figure 2 demonstrates the sealing device (1) in perspective.
Figure 3 shows a section of the sealing device and its details A and B.
Figure 4 shows the *occluder clamp* device.
Figure 5 reveals the side view of the *occluder clamp* device, showing its fins through detail C.
Figure 6 shows the *occluder clamp* device in a new perspective, highlighting the support for fitting the sealing device through detail D.
Figure 7 shows a new side view of the *occluder clamp* device, and highlights the various stages for adjusting the degree of occlusion in detail E.
Figure 8, from a posterior view of the *occluder clamp,* highlights the finger fitting in detail F.
Figure 9 demonstrates the assembly of the kit on the endotracheal tube.
Figure 10 demonstrates the correct direction of the guide wire through the system: tube, connector, sealing device, and *occluder clamp* device.
Figure 11 shows the complete system assembled and prepared for the intubation procedure.

### DETAILED DESCRIPTION OF THE INVENTION

The present invention describes an Endotracheal Tube Barrier Kit with sealing cap attached to *clamp* for use in advanced airway management procedures consisting of two devices, one for sealing and one for "clamping" the tube, coupled together. The kit consists of the following components: sealing device (1) and *occluder clamp (2).* Illustratively, to understand more clearly the use of the Barrier Kit, the tube devices (3) are also shown, connector (4) and guide wire (5), which are components to which the barrier kit is applied.

The first device in the barrier kit is the device for sealing (1) gases, aerosols, droplets, and secretions from the endotracheal tube. Such a sealing device is composed of flexible material, preferably selected from the group containing flexible elastomer material, latex, rubber, or flexible polyurethane, ensuring elastic properties that are fundamental to sealing. With a circumferential shape of about 20mm in diameter and approximately 10mm high in the central part, it has a virtual central hole (A) for the passage of the guide wire (5) identified with a relief different from the main body to facilitate its identification, and a flap, called the removal ear, made of the same material, to facilitate the removal, extraction, of the device during intubation.

The sealing device (1) is used attached to the larger hole in the connector for the mechanical ventilation circuit present on the tubes and/or probes and/or endotracheal catheters, and a second device for "clamping" or occluding the tubes and/or probes and/or endotracheal catheters for gases, aerosols, droplets and patient secretions, which may be used on the body of the tubes and/or probes and/or endotracheal catheters attached near the connector for the mechanical ventilation circuit or away from it.

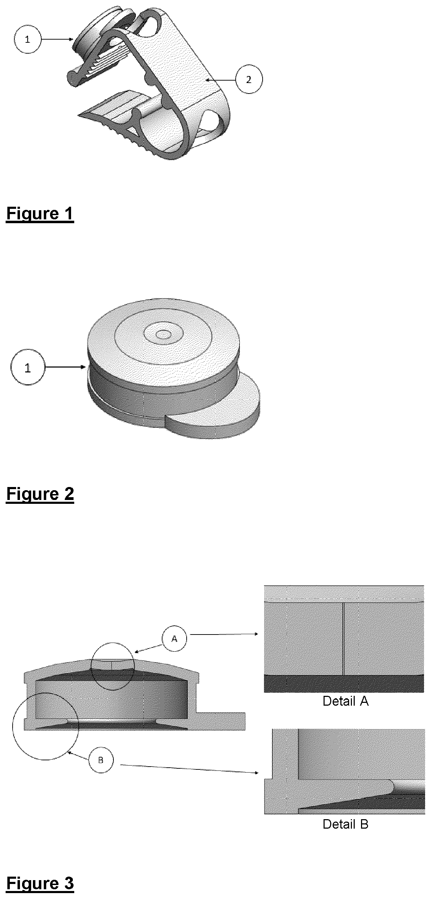

Figure 1 shows the first device, sealing device (1) attached to the second device, *occluder clamp* device (2).

Figure 2 shows a perspective image of the sealing device (1).

Figure 3 shows a sectional view of the sealing device (1). Its elastic composition allows its body to be deformed in such a way as to allow the guide wire, of greater thickness, to penetrate through the central virtual orifice (A) (Detail A), sealing the exit of gases, aerosols, droplets, and secretions by the elastic pressure that the material makes against the wall of the guide wire. Its elastic composition is also used to promote the sealing of the connector by deformation of the inner walls (B) (Detail B). When the sealing device (1) is fitted to the connector, the inner walls (B) deform and press against the connector wall, thus sealing takes place.

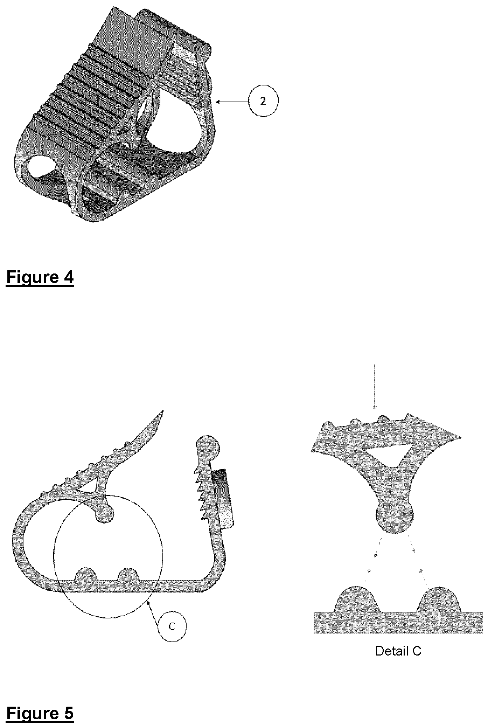

The second device, called the endotracheal tube *occluder clamp* (2) (Figure 4), is confectioned in a single body with resistant plastic material, preferably selected from the group containing poly lactic acid plastic (PLA) and high density polyethylene (HDPE), presenting central holes through which the endotracheal tube passes, and having a fin (C) with a cylindrical shape on one of its sides, in order to be fitted into a gap formed by two other fins (C), also cylindrical, on the opposite side. This structuring allows atraumatic "clamping" of the tube, with a greater deformation angle than that of the tube, with the possibility of sealing up to 100% of its lumen when in use, as shown in Figure 4.

Figure 5 shows the side view of the *occluder clamp* device (2), highlighting the fins (C) (Detail C) used to press the tube and cause obstruction to the passage of gases, aerosols, droplets, and secretions inside the tube. After actuating the device, the force is directed through the upper fin (C) by pressing the tube into the two fins (C) at the bottom, promoting a larger area of obstruction within the tube, the arrows in Detail C indicate the direction of the forces on the fins (C).

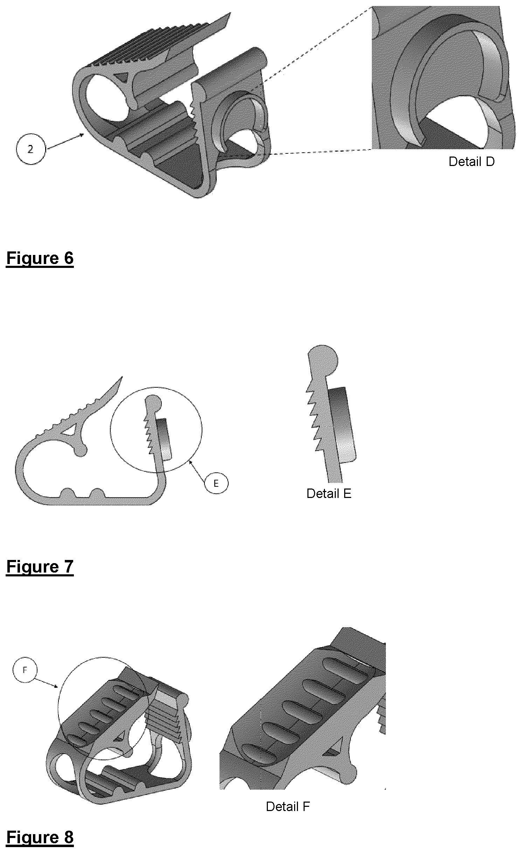

Figure 6 shows the *occluder clamp* device (2) in a new perspective, highlighting the support for fitting (D) of the sealing device (1) (detail D) to allow the two devices to connect and form the endotracheal tube barrier kit with sealing cap attached to the *clamp* for use in advanced airway management procedures.

Figure 7 shows a new side view of the *occluder clamp* device, and highlights the various stages for adjusting the degree of occlusion (E) (detail E), which allows its more adequate and precise use in the various existing pipe diameters, and enables its correct adjustment until the complete occlusion of the pipe when necessary.

Figure 8, from a posterior view of the *occluder clamp,* highlights the finger fitting (F) (detail F), This makes the device more ergonomic and allows for more comfort, and improves the mechanics of its use.

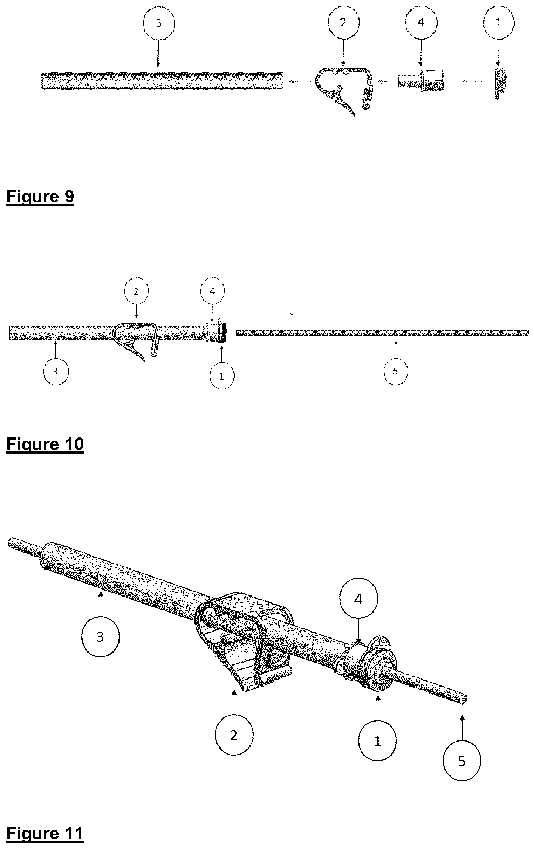

Figures 9, 10 and 11 show how the Barrier Kit for gases, aerosols, droplets and secretions on the endotracheal tube for advanced airway management should be assembled.

Figure 9 demonstrates the assembly of the kit on the endotracheal tube. With the tube assembly (3) and endotracheal tube connector (4) disassembled and the devices in the kit disconnected, use the two center holes at the ends of the *occluder clamp* (2) to pass the tube (3) through its body, positioning the tube between the fins (C) of the device. The connector (4) is normally made of plastic material and has a diameter of approximately 15mm as standard.

Then the smallest hole of the connector (4) is pressed against the tube (3), and the parts are joined together. Finally the sealing device (1) is placed in the larger hole of the connector (4). The arrows indicate the direction of placement of the parts.

Figure 10 shows the correct direction of flow of the guide wire (5) through the system: tube (3), connector (4), sealing device (1) and *occluder clamp* device (2).

The sealing device (1) contains a recess that highlights the central part where it contains the virtual hole for the guide wire (5) to pass through, as shown in Figures 2 and 3. The guide wire is used to help obtain a better positioning of the tube in the patient's airway, even facilitating its identification; it is about 5mm in diameter and may be composed of several materials.

Figure 11 shows all the device parts - (1) and (2) - fixed in their positions, demonstrating the complete system assembled and prepared for the intubation procedure - including the tube devices (3), connector (4) and guide wire (5), not belonging to the set of the proposed invention.

### REFERENCE MARKS

- 1-: sealing device;
- 2-: *occluder clamp;*
- 3-: pipe;
- 4-: connector;
- 5-: guiding thread;
- A-: central virtual hole;
- B-: internal walls;
- C-: fin;
- D-: support for fitting;
- E -: several stages for adjusting the degree of occlusion;
- F-: finger fitting.

## Claims

1. Endotracheal tube barrier kit with sealing cap attached to *clamp* with several stages for adjusting the degree of occlusion (E) for use in advanced airway management procedures **FEATURED** by the fact that it comprises:
a sealing device (1) with a circumferential shape with a central virtual hole (A) made of flexible material with the possibility of deformation of its internal walls (B) and a removal ear, made of the same flexible material as the said device; and
an *occluder clamp* (2), being a single-body structure composed of plastic material that has central holes through which the endotracheal tube passes, a support for fitting (D) of the sealing device (1), a finger attachment (F), having a fin (C) with a cylindrical shape on one of its sides, so as to be fitted into a gap formed by two other fins (C), also cylindrical, on the opposite side.
